(19) **Europäisches Patentamt**

**European Patent Office**

**Office européen des brevets**

(11) **EP 1 065 602 B1**

(12) **EUROPÄISCHE PATENTSCHRIFT**

(45) Veröffentlichungstag und Bekanntmachung des Hinweises auf die Patenterteilung:
**26.05.2004 Patentblatt 2004/22**

(51) Int Cl.⁷: **G06F 17/00**, A61B 5/02

(21) Anmeldenummer: **00112929.5**

(22) Anmeldetag: **20.06.2000**

(54) **Verfahren zur Bestimmung der Baroreflex-Latenzzeit und Baroreflex-Sensitivität**

Method for determining the Baroreflex latency time and the Baroreflex sensitivity

Méthode pour déterminer le temps de latence Baroréflexe et la sensitivité Baroréflexe

(84) Benannte Vertragsstaaten:
**AT BE CH CY DE DK ES FI FR GB GR IE IT LI LU MC NL PT SE**

(30) Priorität: **29.06.1999 DE 19929705**

(43) Veröffentlichungstag der Anmeldung:
**03.01.2001 Patentblatt 2001/01**

(73) Patentinhaber: **BIOTRONIK Mess- und Therapiegeräte GmbH & Co Ingenieurbüro Berlin 12359 Berlin (DE)**

(72) Erfinder: **Meyer, Wolfgang 91052 Erlangen (DE)**

(74) Vertreter: **Hübner, Gerd, Dipl.-Phys. et al Rau, Schneck & Hübner Patentanwälte Königstrasse 2 90402 Nürnberg (DE)**

**Beschreibung**

Die Erfindung betrifft ein Verfahren zur Bestimmung der Baroreflex-Latenzzeit und insbesondere der entsprechenden Baroreflex-Sensitivität.

Der Test der Baroreflex-Sensitivität (BRS) stellt ein seit vielen Jahren gebräuchliches Verfahren dar, mit dem im Rahmen klinischer Untersuchungen die Funktion des Herz-Kreislauf-Systems geprüft wird (s. Liste des Standes der Technik Nr. [4]). So wird z. B. der Test zur Risikostratifizierung von Patienten mit akutem Myokardinfarkt eingesetzt (s. Liste des Standes der Technik Nr. [5]). Die BRS ist das Verhältnis des resultierenden Herzratenabfalls zu einem durch Medikamente hervorgerufenen arteriellen Blutdruckanstieg. Das Verfahren arbeitet damit invasiv und ist nicht belastungsfrei für den Patienten durchführbar. Zudem erfordert die Anwendung ähnlich dem Valsalva-Manöver oder dem aufgrund seiner geringen Spezifität umstrittenen Unterdruckverfahren (s. Liste des Standes der Technik Nr. [8]) immer einen äußeren Eingriff durch den Arzt, und kann somit nur im Rahmen einer Nachsorge erfolgen. Ein spezielles Auswertungsverfahren des BRS-Tests liefert auch Rückschlüsse über die Latenzzeit (s. Liste des Standes der Technik Nr. [6]), d. h. die Dauer zwischen einer Blutdrucksenkung und der durch den Baroreflex vermittelten Rückantwort des Sinusknotens, ist aber kaum gebräuchlich. Eine direkte Bestimmung der Latenzzeit aus nervalen Messungen als exakte Methode (s. Liste des Standes der Technik Nr. [7]) ist in hohem Maß invasiv und damit für die klinische Diagnostik ungeeignet. In jüngerer Zeit erfolgt eine indirekte Einbeziehung der Baroreflex-Funktion durch Frequenzanalyse der Herzratenvariabilität, die jedoch nur den tonischen Teilaspekt der vagalen Aktivität erfaßt (s. Liste des Standes der Technik Nr. [9]), oder durch Analyse der Heart-Rate-Turbulence (s. Liste des Standes der Technik Nr. [10]). Beide Verfahren sind zwar von hohem prognostischem Wert, liefern jedoch kein direktes Maß zur Beschreibung des Baroreflex.

Andererseits hat die Untersuchung gekoppelter Systeme im Bereich der nicht-linearen Dynamik in den letzten Jahren zahlreiche Methoden und Modelle zur Charakterisierung von Systemen mit immanenter Zeitverzögerung bereitgestellt (s. Liste des Standes der Technik Nr. [11], [12]). So ist es möglich durch Messung einer kontinuierlichen Variable auch mehrdimensionale Systeme zu rekonstruieren (s. Liste des Standes der Technik Nr. [3]). Liegt jedoch die Information über ein System als Intervalldaten vor, so ist bislang lediglich eine partielle Rekonstruktion des Phasenraumes möglich, die nur bei streng deterministischen Systemen und genügend langer Meßdauer im stationären Zustand erfolgen kann (s. Liste des Standes der Technik Nr. [2]). Wesentliche Informationen über funktionale Zusammenhänge oder eine immanente Zeitverzögerung werden nicht geliefert.

Daneben existieren zahlreiche Modelle, die eine durch den Baroreflex modulierte Variabilität der Herzrate simulieren (s. Liste des Standes der Technik Nr. [13], [14]). Eines dieser Modelle ist besonders hervorzuheben, da es der Anforderung genügt, auf einfache Weise die Verzögerungszeit des Baroreflex zu berücksichtigen (s. Liste des Standes der Technik Nr. [1]). Das Modell zeigt in Abhängigkeit der Zeitverzögerung qualitativ unterschiedliches dynamisches Verhalten, wobei periodische, komplex periodische und chaotische Domänen auftreten.

Damit stehen zwei wesentliche Hilfsmittel zur Untersuchung neuer Charakterisierungsmethoden des Baroreflex zur Verfügung. Zum einen ist es möglich, ein realistisches aber einfaches Modell so einzustellen, daß die durch Latenzzeitänderungen hervorgerufenen dynamischen Prozesse qualitativ weitestgehend verändert werden können. Zum anderen liefern Methoden der nicht-linearen Dynamik direkte Instrumente zur Rückrechnung eingestellter Modellparameter ausschließlich aus generierten Signalen.

Auf der Basis dieser Ansätze liegt der Erfindung nun die Aufgabe zugrunde, eine nicht-invasive und damit schonende automatische Untersuchung und Bestimmung der Baroreflex-Latenzzeit und entsprechend der Baroreflex-Sensitivität insbesondere anhand des Karotissinus-Reflexes zu ermöglichen.

Ein Verfahren gemäß dem Oberbegriff des Anspruchs 1 ist aus US-A-52011321 bekannt.

Erfindungsgemäß ist bei dem Verfahren zur Bestimmung der Baroreflex-Latenzzeit und entsprechend der Baroreflex-Sensitivität vorgesehen, Herz-Intervall-Dauern, wie das PP-, PR- oder RR-Intervall, nicht-invasiv zu messen und die gemessenen Intervall-Dauern auf der Basis nicht-linearer dynamischer Verfahren einer mathematischen Analyse zu unterwerfen, die folgende Verfahrensschritte aufweist:

- Interpolation der Intervall-Meßwerte zur Ermittlung eines kontinuierlichen Meßwertverlaufs aus den diskreten Intervall-Dauern,
- Auftragen der interpolierten Signalwerte in einem dreidimensionalen Phasenraum mit den Abszissen $y(t)$, $y(t-\tau)$ und $d(y)/d(t)$, wobei für den Parameter $\tau$ unterschiedliche Baroreflex-Latenzzeiten angenommen werden,
- Projektion der dreidimensionalen Auftragung in den zweidimensionalen Raum durch Ermittlung aller Werte auf einer Schnittebene senkrecht zu einer der Achsen, wobei vorzugsweise $y(t)$=konstant gilt, und
- Ermittlung derjenigen angenommenen Baroreflex-Latenzzeit als zutreffend, für die in der zweidimensionalen Projektion die größte Ordnung feststellbar ist.

Die Überprüfung darauf, ob die zweidimensionale Projektion eine Ordnung aufweist, läuft auf das Auffinden einer Linie bzw. eines Verlaufs im Gegensatz zu einer "Punktwolke" hinaus. Zu diesem Zweck werden die Abstände zwischen zu einer Koordinaten-Achse benachbarten Punkten aufsummiert, wobei minimaler Summenabstand maximale Ordnung bedeutet.

Das vorgeschlagenen Verfahren ist durch die Projektion auf den zweidimensionalen Raum stark vereinfacht, wobei die Projektion eine Art Skalierung darstellt. Es könnte jedoch auch ein nicht-skalarer, mehrdimensionaler Ansatz vorteilhaft eingesetzt werden. Allerdings ist dieser mit einem sehr viel höheren Rechenaufwand verbunden.

Die Erfindung wird nachfolgend anhand der beigefügten Zeichnungen näher erläutert. Es zeigen:

Fig. 1    Schaubild eines sogenannten Windkesselmodells, wie es als Basis für das erfindungsgemäße Verfahren benutzt wird,

Fig. 2    ein Kurvenschaubild zur Darstellung des Verlaufes der instantanen Herzperiode T(t) bei einer Verzögerungszeit von t=2,5 s,

Fig. 3    ein Kurvenschaubild zur Darstellung der Polygonlänge L in Abhängigkeit der angenommenen Verzögerungszeiten t bei einer tatsächlichen Verzögerungszeit von τ=2,5 s,

Fig. 4    ein Kurvenschaubild zur Darstellung der Übereinstimmung zwischen theoretisch vorgegebener und experimentel-numerisch bestimmter Verzögerungszeit,

Fig. 5    ein Kurvenschaubild zur Darstellung des berechneten time-delay nach Triggerung des Baroreflex-Modells mittels integrate-and-fire-Methode und Bestimmung der instantanen Herzperiode mittels linearer Interpolation der PP-Intervalle,

Fig. 6    ein Kurvenschaubild zur Darstellung des Spektrums der Polygonlänge in Abhängigkeit der angenommenen Verzögerungszeit für eine Zeitreihe intrakardialer PP-Intervalle bei einem Patienten ohne atriale Extrasystolen, und

Fig. 7    ein Kurvenschaubild zur Darstellung des Spektrums der Polygonlänge in Abhängigkeit der angenommenen Verzögerungszeit für eine Zeitreihe intrakardialer PP-Intervalle bei einem Patienten mit PAF (nicht akut) während einer Periode frei von Extrasystolen.

Im folgenden wird zunächst das Modell vorgestellt und das Rekonstruktionsverfahren näher beschrieben. Danach wird die Rekonstruktion der Zeitverzögerung des Modells diskutiert und das Modell auf einfache Weise in ein getriggertes "beat-to-beat-Modell" überführt, das ebenfalls rekonstruiert wird. Das Verfahren wird anschließend auf intrakardiale Meßdaten angewendet. Schließlich erfolgt eine Diskussion der Ergebnisse und ein Ausblick auf weitere Ausarbeitungen der hier vorgestellten Ansätze, insbesondere hinsichtlich einer automatischen, nicht-invasiven Bestimmung der BRS zusätzlich zur Latenzzeit.

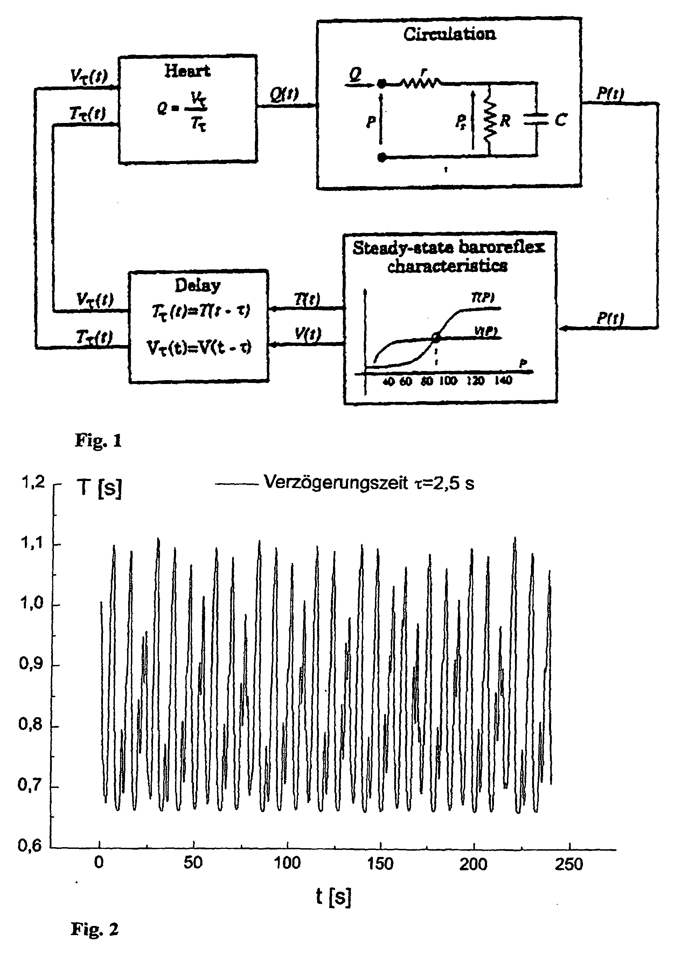

Grundlage des Modells ist ein einfacher Windkessel-Ansatz (Fig. 1). Er führt zu den Gleichungen:

$$\frac{dP_s(t)}{dt} = \frac{1}{RC}[RQ(t) - P_s(t)]$$

$$P(t) = P_s(t) + rQ(t)$$

$$T(P) = T_s + \frac{T_m - T_s}{1 + \gamma e^{-\alpha P/P_n}}$$

$$V(P) = \frac{V_{max}}{1 + \beta\left(\frac{P}{P_v} - 1\right)^{-k}}$$

Die Modellkonstanten werden nach [1] aus physiologischen Meßwerten bestimmt. Die Gleichungen werden nach dem Euler-Verfahren mit Schrittweiten von 0,01 integriert, wobei für Reihenuntersuchungen die Zeitverzögerung in Schritten von 0,1 s zwischen 1,5 s und 3,0 s variiert wird um Zeitreihen mit verschiedener Verzögerungskonstante zu erhalten. Die Integration liefert das Signal eines instantanen Intervalles T(t) eines instantanen Schlagvolumens V(t) und eines instantanen Druckes P(t). Ein Beispiel für einen berechneten Verlauf von T(t) mit t=2,5 s ist zum Vergleich mit [1] nach einer Einschwingzeit in Fig. 2 dargestellt.

Das Modell zeigt laut [1] für den verwendeten Parameterbereich chaotisch-deterministisches Verhalten, durch eine strenge Analyse belegt ist dies jedoch nicht. Das Modell bildet auch komplex-periodische Oszillationen und ansatzweise, trotz kontinuierlicher Herzperiode, auch das Frequenzspektrum einer üblichen Zeitreihe von PP- oder RR-Intervallen nach. Alle verwendeten Parameter bis auf die Latenzzeit sind auf physiologische Messungen zurückgeführt. Die Latenzzeit im chaotischen Teilraum des Parametersatzes wird typisch zwischen 2 s und 3 s angesetzt, darunter wird eine Kaskade von Periodenverdopplungen angenommen. Der Ansatz der Latenzzeit erscheint mit Blick auf die bestehende Literatur nicht haltbar, ist jedoch für das hier vorgestellte Verfahren sekundär. Ebenso zweitrangig ist die Frage, in welchem Maß die hier verwendete Form des Modells einen wichtigen Faktor darstellt. Entscheidend für das folgende Rekonstruktionsverfahren ist lediglich die Zeitverzögerung, nicht aber der explizite funktionelle Zusammenhang der beteiligten Größen, solange die Kopplung gegeben ist.

Das Rekonstruktionsverfahren folgt im wesentlichen den in [3] und anderen Veröffentlichungen der gleichen Autoren skizzierten Prinzipien. Ausgangspunkt der Überlegungen ist die Annahme eines zeitverzögerten Differentialgleichungssystem der Form

$$\dot{y}(t) = h(y(t), y(t - \tau)),$$

wobei hier zunächst von einem Freiheitsgrad y ausgegangen wird. Das aufgrund der Zeitverzögerung unendlich-dimensionale System existiert als Projektion auf einen dreidimensionalen Phasenraum

$$\left( y(t - \tau), y(t), \dot{y}(t) \right).$$

Durch y(t)=c wird das dreidimensionale System auf eine zweidimensionale Ebene projiziert, d. h. es wird ein Poincaré-Schnitt durchgeführt. In die Topologie einer auf diese Weise experimentell bestimmten Punktmenge

$$\left( y(t - \tau), c, \dot{y}(t) \right)$$

geht die Verzögerungszeit t ein. Über diese unbekannte Verzögerungszeit wird jeweils bei der Rekonstruktion eine Annahme getroffen. Abhängig von dem gewählten t ergibt sich eine Struktur der Punktmenge in der Ebene, die um so unregelmäßiger ist, je weiter die gewählte von der tatsächlichen Verzögerungszeit oder von deren Vielfachen abweicht. Die Struktur läßt sich z. B. durch den Füllfaktor (Anteil der mit Punkten besetzten Phasenraumzellen) oder durch einfachere Ansätze wie die Messung der Länge des die Punktmenge verbindenden Polygonzuges charakterisieren.

Das Prinzip läßt sich grundsätzlich auch auf Systeme mit mehr als einem Freiheitsgrad wie das vorliegende Baroreflex-Modell anwenden. Das vorstehend beschriebene Modell läßt sich auf die o. a. verwendete Form bringen und ist damit der beschriebenen Analyse zugänglich. Die genaue Implementierung hängt entscheidend von der Form des Systems und der Anzahl der Freiheitsgrade ab. Da jedoch zunächst nur die Latenz bestimmt werden soll, wird in dieser Untersuchung jeweils ein skalarer Ansatz verfolgt, was auch deshalb sinnvoll erscheint, weil bei späteren Anwendungen nur eine Zustandsvariable, nämlich die Herzperiode auch tatsächlich zur Verfügung steht.

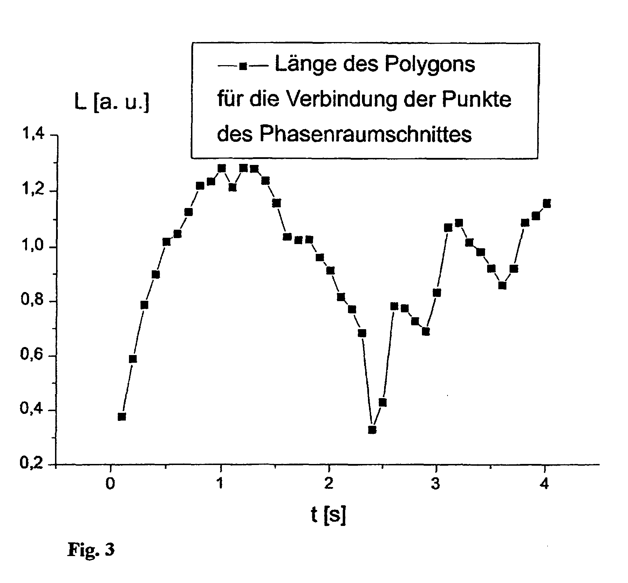

Die Rekonstruktion der Zeitverzögerung stellt sich wie folgt dar:
Zunächst werden nach dem vorstehend erläuterten Verfahren Signalverläufe mit verschiedenen Verzögerungszeiten von 1,5 s bis 3,0 s generiert. Mit der beschriebenen Vorgehensweise werden unter Verwendung des Polygonverfahrens alle Zeitreihen analysiert. Ein typischer Verlauf des resultierenden Profils der Polygonlänge in Abhängigkeit der angenommenen Verzögerungszeit ist in Fig. 3 für die Verzögerungszeit t=2,5 s dargestellt.

Die Verzögerungszeit läßt sich aufgrund des festgestellten Profils durch Annahme des absoluten Minimums bei der tatsächlichen Verzögerungszeit zu t=2,4 s bestimmen. Der Fehler von 0,1 s resultiert im wesentlichen aus der Unschärfe von 0,1 s bei der Rekonstruktion. Der Fehler läßt sich durch höhere Auflösung bei der Rekonstruktion bzw. durch die Berücksichtigung der gesamten Peakbreite z. B. durch eine parabolische Funktionsanpassung weiter reduzieren.

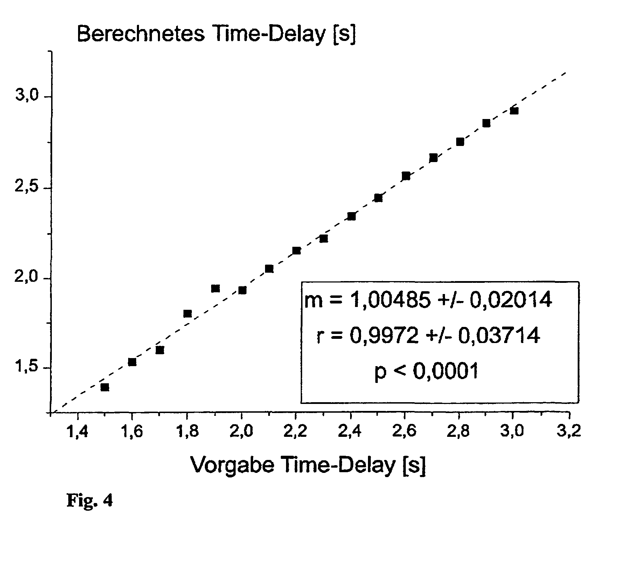

Die Korrelation zwischen der tatsächlichen Verzögerungszeit und der mit Hilfe der oben beschriebenen einfachen Minimalverfahren ermittelten Verzögerungszeit ist in Fig. 4 aufgetragen. Man erkennt eine weitgehende Übereinstimmung von theoretischer Vorgabe und experimentell-numerisch bestimmtem Wert. Der Korrelationskoeffizient ist ebensowenig von 1 unterscheidbar wie die Steigung der Regressionsgeraden. Die Korrelation ist hochsignifikant.

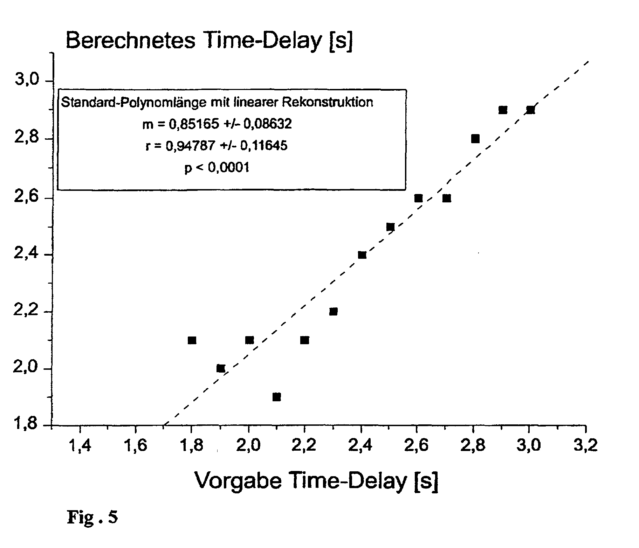

Die Rekonstruktion im beat-to-beat Modell stellt sich nun wie folgt dar:
Die vorstehend erläuterte Rekonstruktion erfolgte unter Verwendung des kontinuierlichen Signalverlaufs. Dieser steht jedoch bei Messung der Herzperiode nicht zur Verfügung. Statt dessen handelt es sich bei der Bestimmung der Intervalle am Herzen um die Beobachtung einer getriggerten Aktivität. Deshalb wurde zunächst das kontinuierliche Modellsignal in eine Zeitreihe der PP-Intervalle transformiert. Hierzu wurde ein einfaches integrate-and-fire Modell angesetzt, wobei die reziproke instantane Herzperiode als zeitabhängiger Schwellwert interpretiert wurde. Um das Rekonstruktionsverfahren auch auf die generierten Intervalle anwenden zu können, wurde der Weg der Interpolation beschritten, d. h. eine instantane Herzperiode wurde durch lineare Interpolation zwischen den PP-Intervallen erhalten. Mit der so generierten Zeitreihe, die wie das nicht getriggerte Originalsignal eine Abtastrate von 100 Hz aufweist, wurde ohne weitere Änderungen des algorithmischen Ablaufs der Parameter der Polynomlänge im Phasenraum für verschiedene Annahmen der Verzögerungszeit bestimmt. Die Ergebnisse für die verschiedenen Verzögerungszeiten sind in Fig. 5 aufgetragen.

Vorgegebene und experimentell-numerisch berechnete Zeitverzögerungen sind auch bei der linearen Interpolation der PP-Intervalle nach vorheriger Triggerung des kontinuierlichen Baroreflex Modells mittels integrate-and-fire noch hoch-signifikant korreliert. Lediglich bei Werten von t=1,8 s und t=2,1 s ist die Bestimmung der Verzögerungszeit mit einem Fehler von 0,3 s bzw. 0,2 s erfolgt, für die Vorgaben 1,5 s bis 1,7 s war keine eindeutige Identifikation einer Verzögerungszeit aus dem Spektrum der Polynomlängen erkennbar. Insbesondere hier bieten sich weitere Ansätze zur Verbesserung des Verfahrens an, z. B. durch Implementierung eines nicht-linearen Interpolationsverfahrens.

Entscheidend für die Anwendbarkeit des Rekonstruktionsverfahrens ist letztlich nicht die Rekonstruierbarkeit von Parametern der Simulation, sondern die Bestimmbarkeit einer Latenzzeit aus kardiologischen Meßdaten, die zudem mit physiologischen Gegebenheiten korreliert. In den folgenden Absätzen werden daher mit dem einfachen Verfahren erhaltene Ergebnisse demonstriert, die anhand intrakardialer Meßreihen erhalten wurden.

Die Anwendung des theoretisch erprobten Verfahrens stellt einen weitgehend empirischen Vorgang dar. Hinzu kommt, daß über die Bedeutung der Latenzzeit im Gegensatz zur BRS nur gelegentliche Vermutungen im Zusammenhang mit pathologischen Veränderungen geäußert werden [6]. Der Vergleich der beiden folgenden Zeitreihen muß deshalb unter dem Vorzeichen der beschränkten, erst in Ansätzen vorliegenden Erkenntnisse über die Realisierbarkeit des Rekonstruktionsverfahrens gesehen werden.

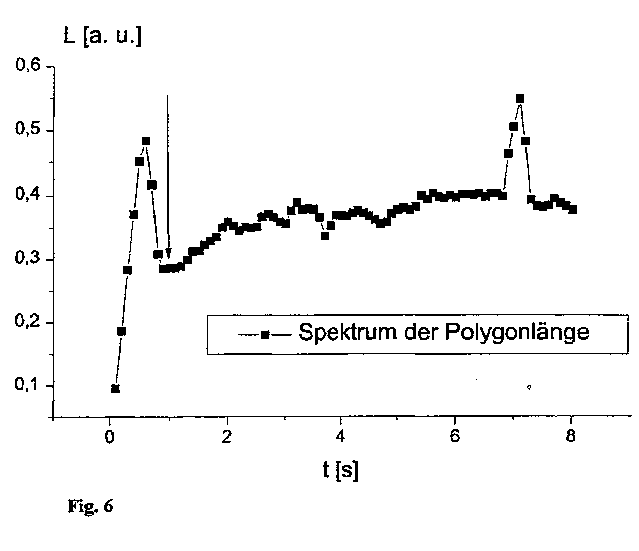

Die erste Zeitreihe wurde durch Messung intrakardialer EKG-Signale erhalten. Es erfolgte eine Triggerung des atrialen EKG auf die P-Zacke, wonach die erhaltenen PP-Intervalle Gegenstand von Interpolation und Rekonstruktion waren. Die Meßdauer betrug hierbei ca. 2 h und es ist von zahlreichen Nicht-Stationaritäten des Herz-Kreislauf-Systems auszugehen. Das Spektrum der Polygonlängen für die gesamte Zeitreihe ist in Fig. 6 aufgetragen.

Die Zeitreihe ist nach Interpretation des Spektrums der Polygonlängen mit einem System von Differentialgleichungen verträglich, das eine Verzögerungszeit von 1,0 s aufweist. Das eindeutig identifizierte Minimum des Spektrums ist durch einen Pfeil markiert.

Die zweite Zeitreihe wurde über einen ähnlich langen Zeitraum (ca. 2 h) aufgezeichnet. Der Patient leidet an paroxysmalem Vorhofflimmern (PAF). Die Messung erfolgte in einem Zeitabschnitt, in dem keine atrialen Extrasystolen vorkamen, nach dem ausgewerteten Abschnitt aber gehäuft auftraten. Flimmern trat zum Zeitpunkt der Aufzeichnung nicht auf.

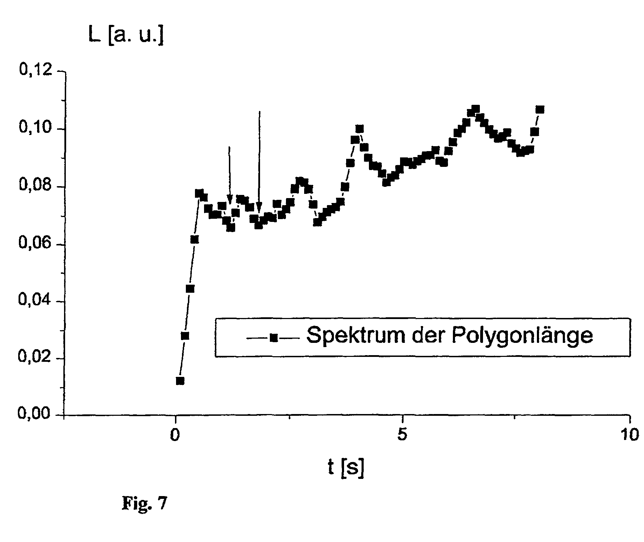

Die eindeutige Interpretation im Hinblick auf eine Verzögerungszeit fällt im Fall von Fig. 7 schwer. Zwar ist das erste und mit geringem Abstand tiefste Minimum bei 1,2 s angesiedelt (kurzer Pfeil), doch ist die Breite des zugeordneten Troges anders als in Fig. 6 kaum ausgeprägt. Das zweite Minimum (langer Pfeil) bei 1,8 s ist dagegen breit ausgeprägt Hinzu kommt, daß Minima höherer Ordnung etwa bei 3,4 s und 4,9 s ebenfalls auftreten, so daß eine Verzögerungszeit von t=1,7 s konsistent anzunehmen ist. Das Ergebnis läßt sich somit als Tendenz interpretieren, bei erhöhter Anfälligkeit für PAF oder Extrasystolen eine Verlängerung der Latenzzeit festzustellen. Das Beispiel zeigt, daß physiologische Zeitreihen erwartungsgemäß schwieriger zu interpretieren sind, als numerische Simulationen. Aus den Unterschieden zwischen Fig. 6 und Fig. 7 lassen sich bereits weitere Ansatzpunkte für die Interpretation und Verbesserung des Verfahrens ableiten.

So zeigt sich in der Mehrdeutigkeit von Fig. 7 einerseits eine Instabilität, die auf pathologisch bedingte Schwankungen der Latenz zurückzuführen sein kann. Die schwache Ausprägung eines Hauptminimum läßt sich dabei als Kennzeichen einer geringen Baroreflex-Sensitivität interpretieren, die sich auch in einer fehlenden zeitlichen Korrelation und geringen Herzratenvariabilität ausdrückt, wie sie mit herkömmlichen Verfahren bei der gleichen Zeitreihe detektiert werden.

Grundsätzlich sind beide Ergebnisse vorsichtig zu interpretieren, solange das Verfahren nicht einer umfassenden Validierung unterzogen wurde. Dies kann neben der Verfeinerung von Modellvorstellungen und der Anpassung bestehender Systeme nur dadurch geschehen, ein physiologisches Korrelat, d. h. eine bestimmte Menge von Erkrankungen bzw. Vergleichsgrößen aufzufinden, die bei strengen Kriterien für die Bestimmung der Latenzzeit mit dieser eine Beziehung herstellen.

Zusammenfassend ist folgendes festzuhalten: Anhand eines kardiovaskulären Modellsystems des Baroreflex mit Verzögerung und eines Verfahrens zur Rekonstruktion dieser Verzögerungszeit wurde gezeigt, daß die aus der nicht-linearen Dynamik bekannten Methoden sich auf das Modellsystem anwenden lassen. Dies gilt auch noch für die Rekonstruktion getriggerter Zeitreihen. Eine systematische Verbesserung des Verfahrens und seine Anwendung auf umfangreiche kardiologische Zeitreihen ist anzustreben.

Folgende Literaturstellen sind als Stand der Technik anzugeben:

[1] Cavalcanti, S., Belardinelli, E., Modeling of Cardiovascular Variability Using a Differential Delay Equation, IEEE Trans. Biomed. Eng. 43: 982-989 (1996)

[2] Sauer, T., Reconstruction of Dynamical Systems from Interspike Intervals, Phys. Rev. Lett. 72: 3811-3814 (1994)

[3] Bünner, M. J., Meyer, Th., Kittel, A., Parisi, J., Recovery of the time-evolution equation of time-delay systems from time series, Phys. Rev. E 56: 5083-5089 (1997)

[4] LaRovere, M. T., Mortara, A., Schwartz, P. J., Baroreflex Sensitivity, J. Cardiovasc. Electrophysiol. 6: 761-774 (1995)

[5] LaRovere, M. T., Bigger, J. Th., Marcus, F. I., Mortara, A., Schwartz, P. J., Baroreflex sensitivity and heart-rate variability in prediction of total cardiac mortality after myocardial infarction, Lancet 351: 478-484 (1998)

[6] Smith, St. A., Stallard, T. J., Littler, W. A., Estimation of sinoaortic baroreceptor heart rate reflex sensitivity and latency in man; a new microcomputer assisted method of analysis, Cardiovasc. Res.

20: 877-882 (1986)

[7] Fagius, J., Sundlöf, G., Wallin, B. G., Variation of sympathetic reflex latency in man, J. Auton. Nerv. Syst. 21: 157-165 (1987)

[8] Potts, J., Raven, P. B., Effect of dynamic exercise on human carotid-cardiac baroreflex latency, Am. J. Physiol. 268: H1208-H1214 (1995)

[9] DeFerrari, G. M., Landolina, M., Mantica, M., Manfredini, R., Schwartz, P. J., Lotto, A., Baroreflex sensitivity, but not heart rate variability, is reduced in patients with life-threatening ventricular arrhythmias long after myocardial infarction, Am. Heart J. 130: 473-480 (1995)

[10] Schmidt, G., Malik, M., Barthel, P., Schneider, R., Ulm, K., Rolnitzky, L., Camm, A. J., Bigger, J. Th., Schömig, A., Heart-rate turbulence after ventricular premature beats as a predictor of mortality after acute myocardial infarction, Lancet 353: 1390-1396 (1999)

[11] Bünner, M. J., Popp, M., Meyer, Th., Kittel, A., Rau, U., Parisi, J., Recovery of scalar time-delay from time series, Phys. Lett. A 211:345-349 (1996)

[12] Bünner, M. J., Popp, M., Meyer, Th., Kittel, A., Parisi, J., Tool to recover scalar time-delay systems from experimental time series, Phys. Rev. E 54: R3082-R3085 (1996)

[13] DeBoer, R. W., Karemaker, J. M., Strackee, J., Hemodynamic fluctuations and baroreflex sensitivity in humans: a beat-to-beat model, Am. J. Physiol. 253: 680-689 (1987)

[14] Seidel, H., Herzel, H., Modelling Heart Rate Variability due to Respiration and Baroreflex, preprint

## Patentansprüche

1. Verfahren zur Bestimmung der Baroreflex-Latenzzeit, insbesondere der entsprechenden Baroreflex-Sensitivität umfassend

   - nicht-invasives Messen von Herz-Intervall-Dauern und
   - mathematisches Analysieren der Meßwerte der Herz-Intervall-Dauern auf der Basis nicht-linearer dynamischer Verfahren, **dadurch gekennzeichnet, daß** das nichtlineare dynamische Analyseverfahren folgende Schritte aufweist:

     - Interpolation der Intervall-Meßwerte zur Ermittlung eines kontinuierlichen Meßwertverlaufs aus den diskreten Intervall-Dauern,
     - Auftragen der interpolierten Signalwerte in einem dreidimensionalen Phasenraum mit den Abszissen y(t), y(t-τ) und d(y)/d(t), wobei für den Parameter τ unterschiedliche Baroreflex-Latenzzeiten angenommen werden,
     - Projektion der dreidimensionalen Auftragung in den zweidimensionalen Raum durch Ermittlung aller Werte auf einer Schnittebene senkrecht zu einer der Achsen, wobei vorzugsweise y(t)=konstant gilt, und
     - Ermittlung derjenigen angenommenen Baroreflex-Latenzzeit als zutreffend, für die in der zweidimensionalen Projektion die größte Ordnung feststellbar ist.

2. Verfahren nach Anspruch 1, **dadurch gekennzeichnet, daß** als Herz-Intervall-Dauern die PP-, PR- und/oder RR-Intervalle des Herzens nicht-invasiv gemessen werden.

## Claims

1. A method for determining the baroreflex latent period, particularly the corresponding baroreflex sensitivity, comprising

   - the non-invasive measurement of cardiac-interval periods, and
   - the mathematical analysis of the measurement values of the cardiac-interval periods on the basis of non-linear dynamic methods,

     **characterized in that** the non-linear dynamic analysis method comprises the following steps:

     - the interpolation of the interval measurement values for determining a continuous measurement-value course from the discrete interval periods;
     - the graphing of the interpolated signal values in a three-dimensional phase space with the abscissas y(t), y(t - τ) and d(y)/d(t), with different baroreflex latent periods being assumed for the parameter τ;
     - the projection of the three-dimensional graph into a two-dimensional space through the determination of all values on a sectional plane perpendicular to one of the axes, with y(t) preferably being constant; and
     - the determination of the assumed baroreflex latent period for which the highest order can be established in the two-dimensional projection as being relevant.

2. The method according to claim 1, **characterized in that**, as cardiac-interval periods, the P-P, P-R and/or R-R cardiac intervals are measured non-invasively.

**Revendications**

1. Procédé de détermination du temps de latence baroréflexe, notamment de l'excitabilité baroréflexe correspondante, comprenant :

   - un mesurage non invasif des durées d'intervalle cardiaque et
   - une analyse mathématique des valeurs mesurées des durées d'intervalle cardiaque sur la base de procédés dynamiques non linéaires, **caractérisé en ce que** le procédé d'analyse dynamique non linéaire comporte les étapes suivantes :

      - interpolation des valeurs mesurées d'intervalle pour calculer une courbe continue des valeurs mesurées à partir des durées d'intervalle discrètes,
      - report des valeurs de signal interpolées dans un espace de phase tridimensionnel comportant les abscisses y (t), y (t - $\tau$) et d (y) / d(t), différents temps de latence baroréflexes étant admis pour le paramètre $\tau$,
      - projection du tracé tridimensionnel dans l'espace à deux dimensions en calculant toutes les valeurs sur un plan de coupe perpendiculaire à l'un des axes, avec de préférence y (t) = constante, et
      - parmi les temps de latence baroréflexe admis, détermination que le bon est celui pour lequel le plus grand ordre est identifiable dans la projection à deux dimensions.

2. Procédé selon la revendication 1, **caractérisé en ce que** l'on mesure de manière non invasive les intervalles PP, PR et/ou RR du coeur en tant que durées d'intervalle cardiaque.

**Fig. 1**

**Fig. 2**

Fig. 3

**Berechnetes Time-Delay [s]**

m = 1,00485 +/- 0,02014

r = 0,9972 +/- 0,03714

p < 0,0001

**Vorgabe Time-Delay [s]**

Fig. 4

Fig . 5

Fig. 6

Fig. 7